# EUROPÄISCHE PATENTSCHRIFT

(11) **EP 2 568 935 B1**
(45) Veröffentlichungstag und Bekanntmachung des Hinweises auf die Patenterteilung: **22.06.2016**
(21) Anmeldenummer: 11780227.2
(22) Anmeldetag: 10.05.2011
(51) Int. Cl.: A61F 7/02, F28D 20/02, A61F 2/78, A61F 2/80, F28D 20/00

(54) **VORRICHTUNG ZUM TEMPERATURABBAU**
DEVICE FOR TEMPERATURE REDUCTION
DISPOSITIF POUR ABAISSER LA TEMPÉRATURE

(30) Priorität: 11.05.2010 DE 102010020262; 13.01.2011 DE 202011001473 U
(43) Veröffentlichungstag der Anmeldung: 20.03.2013
(73) Patentinhaber: Otto Bock HealthCare GmbH, 37115 Duderstadt (DE)
(72) Erfinder: RADSPIELER, Andreas, 83115 Neubeuern (DE); FIEBACK, Klaus, 14552 Michendorf (DE); BÜTTNER, Dirk Carsten, 12207 Berlin (DE)
(74) Vertreter: Stornebel, Kai
(86) Internationale Anmeldenummer: PCT/DE2011/001050
(87) Internationale Veröffentlichungsnummer: WO 2011/141019

(56) Entgegenhaltungen:
- US-A- 5 115 859
- US-A- 5 386 701
- US-A- 5 415 222
- US-A- 5 722 482
- US-A- 6 010 528
- US-A- 6 047 106
- US-A- 6 125 645
- US-A1- 2003 109 911
- US-A1- 2006 064 147

## Beschreibung

Die Erfindung betrifft eine Vorrichtung zum Temperaturabbau infolge gestauter Körperwärme.

Es ist bekannt, dass an bestimmten Stellen in Gebrauchsgegenständen wie Helmen, Turnschuhen, Autositzen, Fahrradsattel, Reitsattel, sowie in Textilien, also flexiblen Geweben wie Einlagen oder Strümpfen und nicht zuletzt in Körperorthesen oder Körperprothesen sich Körperschweiß vermehrt ansammelt, weil die Erwärmung des Körpers dort intensiv ist und vergleichsweise nur ungenügend abgeführt werden kann, es also zu einer Stauwärme des Körpers an der Stelle im Gebrauchsgegenstand und/oder in dem flexiblen Flächengebilde/der flexiblen Matrix kommt.

Das Problem wird bereits auf vielfältige Weise angegangen, beispielsweise wird bei Sommerschuhen, die geschlossen sein sollen, oben teilweise das Obermaterial, beispielsweise Leder, durch Luftlöcher unterbrochen, so dass sich ein gewisser Luftzug im Schuh ergibt. Die Löcher werden zum Teil durch Netze abgedeckt, so dass die Optik eines geschlossenen Schuhs entsteht. Das gleiche wird bei allen Arten von Helmen praktiziert, wo an schmalen Stellen der Helm einfach unterbrochen wird oder Netze eingebaut werden, damit Kühlluft die Abwärme des Körpers aufnehmen kann. Nachteilig an den Luftlöchern ist, dass sie die Funktion des Helmes an den betroffenen Stellen aussetzen und zum Zweiten, dass an verschiedenen Stellen, beispielsweise und Schuhsohlen oder an Prothesen, eine derartige Unterbrechung des Materials zur Unbrauchbarkeit des gesamten Teils führen würde. Luftlöcher an textilen Oberteilen werden auch durch Reißverschlüsse unter den Achseln bei Jacken etc. angeboten.

Des Weiteren gibt es speichernde Textilien wie z.B. Einlegeschuhsohlen, Bettwäsche etc., die jedoch ohne aktive Wärmeabführung sind.

Aus der US 5,722,482 ist ein textiles Flächengebilde mit einem Phasenwechselmaterial (Phase Change Material, PCM) bekannt. Dieses Flächengebilde ist vorgesehen, um die Körperwärme abzupuffern, das heißt sie dient als Kälteschutz, zum längeren Erhalt der körpereigenen Wärme. Nachteilig an dem aus der US 5,722,482 bekannten flexiblen Flächengebilde ist, dass keine Mittel vorgesehen sind, mit denen die gestaute Körperwärme abführbar ist.

Die US 6,010,528 A betrifft eine Vorrichtung, die zumindest einen Teil eines menschlichen oder tierischen Körpers abstützt und eine Kühlung des gewichttragenden Bereiches des Körperteils bewirkt. Die Vorrichtung weist eine an den Körperbereich angepasste Form auf und ist dazu vorgesehen, Gewebeschäden zu vermeiden oder vorzubeugen, die aufgrund von Druck und Wärme entstehen können. In dem vorgeformten Bereich ist zumindest eine Kühleinrichtung angeordnet, die in einem Ausführungsbeispiel Polster aufweist, unter denen eine Kühlflüssigkeit zirkuliert.

Die US 2003/109911 A1 betrifft eine Kühldecke mit einem Phasenwechselmaterial, das in einer flüssigkeitsundurchlässigen, flexiblen und anpassbaren Umhüllung angeordnet ist. Auf der Außenseite der Umhüllung ist eine Isolierschicht angeordnet, um einen Wärmetransport nach außen zu behindern.

Die US 6 125 645 A betrifft ein Kleidungsstück, mit dem es möglich ist, die Körpertemperatur zu verringern, ohne dass teure Kühlmittel verwendet werden müssen. Das Kleidungsstück nimmt Feuchtigkeit von der Körperoberfläche auf, transportiert diese über ein Kapilarsystem zu einer Wärmesenke und lässt die Feuchtigkeit dort kondensieren. Als Wärmesenke wird z.B. schmelzendes Eis als PCM verwendet.

Aufgabe der Erfindung ist es, eine Vorrichtung zum Temperaturabbau zu schaffen, die in harten, wie beispielsweise Prothesen, sowie in biegsamen, wie beispielsweise Schuhsohlen und in flexiblen, wie beispielsweise Stützstrümpfen, mit dem Körper eines Lebewesens wärmetechnisch in direktem Kontakt stehenden Gebrauchsgegenständen einsetzbar ist, wobei die Vorrichtung in den Gegenstand und/oder die flexible Matrix integrierbar ist, ohne dass die Funktionalität des Gegenstandes und/oder der flexiblen Matrix an der Stelle entfällt.

Die Lösung der Aufgabe wird nachfolgend anhand der Beschreibung, den Figuren und den Ansprüchen offenbart.

Allgemeine Erkenntnis der Erfindung ist, dass mit Hilfe so genannter PCMs (Phase Change Materialien, mit hoher Wärmespeicherkapazität), beispielsweise in Verbindung mit entsprechenden Wärmetauschern an flexiblen und/oder starren Gegenständen, die sich in Körperkontakt befinden, Wärmespeicher bilden lassen, die die Oberflächentemperatur der Haut bzw. der Kontakttemperatur des hautanliegenden Gegenstandes in einem angenehmen Bereich halten und gleichzeitig den Tragekomfort und/oder die Funktionalität des Gegenstandes nicht verändern. Die Regeneration der PCMs erfolgt durch einen Wärmetauscher bevorzugt kontinuierlich, auch während des Tragens.

Der Gegenstand der Erfindung wird durch die Merkmale des Anspruchs 1 definiert, vorteilhafte Ausführungen werden in den abhängigen Ansprüchen beschrieben.

Die erfindungsgemäße Vorrichtung zum Temperaturabbau ist beispielsweise nur in einem Teil des Gebrauchs- oder orthopädischen Gegenstandes oder des weichteilstabilisierenden Gewebes (Strumpfes) enthalten oder realisiert. Der Gebrauchs- oder orthopädische Gegenstand unterscheidet sich vom flexiblen Gewebe vor allem dadurch, dass er entweder eine nichtflexible oder zumindest eine in Teilen feste Form hat. Begrifflich lassen sich die beiden "Kategorien" nicht voneinander trennen, da ein Gegenstand zum Teil eine flexible Form haben kann und ein Gewebe feste Teile. Es ist daher möglich, dass die Vorrichtung gemäß der Erfindung - unter anderem oder nur dort - in einem flexiblen Teilstück eines ansonsten nicht-flexiblen Gegenstandes integriert ist und umgekehrt, dass die Vorrichtung in einem flexiblen Gewebe - unter anderem oder nur dort - an einer Stelle integriert ist, an der sich nicht-flexible Bestandteile befinden. Im Sinne der Erfindung sollen alle diese möglichen Konstruktionen umfasst sein." Nach einer vorteilhaften Ausführungsform hält der Wärmetauscher im Betrieb die Temperatur des PCMs unterhalb und/oder im Schmelzbereich. Damit wird sowohl die Kühlwirkung der PCMs aufrechterhalten, als auch die Temperatur der den Körperteil umgebenden PCMs ungefähr konstant gehalten, so dass keine unerwünschten harten Temperaturschwankungen auftreten.

Erfindungsgemäß ist die Vorrichtung zum Temperaturabbau, insbesondere auch der Wärmetauscher, innerhalb des Gegenstandes integriert, beispielsweise in Form einer Doppelwand, die mit Kühlmedium durchflossen wird oder eines Vlieses mit Rillen, über die Kühlmedium strömt. Ebenso sind die PCMs nicht unbedingt vollflächig vorgesehen, sondern es können einzelne Bereiche des Gegenstandes durch PCM-"Päckchen" ausgerüstet sein, wobei der Wärmetauscher in diesen Fällen vorteilhafterweise auch auf diese Bereiche des Gegenstandes beschränkt sein kann. Zur Integration des PCMs und der Wärmetauscherfunktion in den Gegenständen kann es keine umfassende Beschreibung geben, da die Möglichkeiten sich zum einen nach dem Gegenstand und dann auch nach dem Träger richten.

Nicht erfindungsgemäß ist vorgesehen, dass die Vorrichtung zum Temperaturabbau auch lösbar, also beispielsweise durch einen Gurt oder Klettverschluss gehalten, an dem Gegenstand wie Helm, Schuh, Sattel, befestigt ist. Damit kann je nach Jahreszeit und Bedarf der Gegenstand mit der Vorrichtung nach- und ausgerüstet werden.

Ein Wärmetauscher ist eine komplette Vorrichtung aus wärmeaufnehmender und wärmeabgebender Komponente. Im vorliegenden Fall wird diese Einheit aus PCM-Element und einem vom Wärmeträger durchströmten, am PCM anliegenden Raum gebildet. Die Durchströmung kann im offenen System bzw. als geschlossenes Kreislaufsystem erfolgen. Im offenen System wird das Wärmeträgermedium frei angesaugt, am PCM vorbeigeleitet und dann ins Freie abgegeben. Bei einem geschlossenen System erfolgt die Wärmeabgabe nicht direkt ins Freie, sondern indirekt über einen weiteren, zwischengeschalteten Wärmetauscher.

Beispielsweise können Wärmetauscher, die mit Umgebungsluft betrieben werden, offen betrieben werden. Andererseits werden Wärmetauscher mit speziellen Kühlmedien, wie beispielsweise auch Wasser, bevorzugt geschlossen betrieben.

Die Vorrichtung kann in Form eines flexiblen Flächengebildes, wobei eine flexible Matrix ganz oder teilweise mit PCM oder PCM-haltigen Bereichen versetzt ist, umfassen, wobei Mittel vorgesehen sind, durch die ein Medium eines Wärmetauschers in wärmeübertragenden Kontakt mit den PCMs der flexiblen Matrix gebracht wird, so dass Wärme von den PCMs abgeführt und/oder den PCMs zugeführt werden kann.

Die Mittel - durch die das Medium des Wärmetauschers in Kontakt mit den PCMs treten kann - im Folgenden auch einfach "Mittel" genannt, umfassen Strömungsstrukturen wie Kanäle oder Erhebungen mit verschiedenen Profilen wie Rillen, Kanäle, beispielsweise auch mäanderförmige Kanäle, Noppen, Pyramiden, oder anderes. Das Muster, das sie bilden, kann völlig beliebig sein, es kann auch als Werbeträger dienen. Die Strömungsstrukturen, also die Mittel beeinflussen natürlich die Geschwindigkeit, mit der das Medium über die PCMs strömt.

Nach einer vorteilhaften Ausführungsform der Erfindung bilden die Strömungsstrukturen zumindest teilweise die Oberflächenstruktur einer flexiblen Matrix.

Die Mittel umfassen nach einer weiteren Ausführungsform neben den Strömungsstrukturen auch noch eine Vorrichtung, durch die das Medium des Wärmetauschers bewegt wird.

Nach einer vorteilhaften Ausführungsform liegt das PCM in Form entsprechender Modifikationen eines Paraffins und/oder eines Salzhydrates wie PCM-Polymercompounds, PCM-Silikat-Compounds, PCM-Graphitcompounds usw. vor. In einer weiteren Ausgestaltung können diese PCMs bzw. deren Modifikationen in entsprechende Trägerstrukturen z. B. kapillare Fasersaugstrukturen eingearbeitet sein.

Nach einer vorteilhaften Ausführungsform der Erfindung ist das Wärmeträger-Medium gasförmig, also beispielsweise Umgebungsluft. Dabei kann es vorgesehen sein, dass der Träger des flexiblen Flächengebildes selbst, beispielsweise durch eigene Bewegung (z.B. Schuh, Sattel) die Bewegung des Mediums durch die Strömungsstrukturen zur Abfuhr der Wärme der PCMs realisiert. Andererseits kann auch ein kleiner Ventilator oder eine sonstige Vorrichtung vorgesehen sein, die das Bewegen/Strömen des Mediums bewirkt.

Nach einer vorteilhaften Ausführungsform wird die Strömung des Mediums so eingestellt, dass die Temperatur der PCMs unterhalb oder im Schmelzbereich der PCMs gehalten wird.

Nach einer Ausführungsform der Erfindung sind die Bereiche mit PCMs kleine Inseln in einer flexiblen Matrix, die ein Kühlgewebe oder Kühlvlies bildet.

So werden gemäß einer Ausführungsform in ein am Körper getragenes flexiblen Flächengebilde und/oder Kühlvlies kleine Inseln mit PCM-enthaltenden-Päckchen eingebaut, wobei die Eigenschaften des flexiblen Flächengebildes erhalten bleiben.

Als Material für die flexible Matrix oder Kühlvlies können alle Arten bekannter Polstermaterialien eingesetzt werden. Neben dem bereits erwähnten Silikon sind da beispielsweise noch Polyurethan, thermoplastische Polymere, Copolymere, Polyethylen und andere.

Die Strömungsstruktur kann so gestaltet werden, dass ein der Oberfläche entlang strömendes Medium über die Inseln und/oder Bereiche mit PCMs geleitet wird, so dass dort bevorzugt Wärmeaustausch vom PCM zum Medium stattfindet.

Beispielsweise umfasst eine Unterschenkelprothese in der Regel neben dem festen Schaft auch noch einen flexiblen Silikonstrumpf zur Weichteilstabilisierung, der über den Körperteil gezogen wird und zum einen direkt auf der Haut anliegt und zum anderen in dem Schaft steckt. Das ist beispielsweise ein Silikonstrumpf, der nach einer Ausführungsform der Erfindung, mit Bereichen, beispielsweise mit runden Inseln aus PCM-haltigen Material, versetzt wird, wobei die Eigenschaften des flexiblen Silikonstrumpfes im Wesentlichen erhalten bleiben. Am Schaft kann ein kleiner Ventilator vorgesehen sein, durch den Luft entlang der Innenseite des Schaftes geblasen wird, die dann entlang der Strömungsstrukturen des mit PCM-Bereichen versetzten Silikonstrumpfes strömt. Dabei können die Strukturen, die den Luftstrom lenken, sowohl an der Innenseite des Schaftes als auch an der Außenseite der Einlage, also beispielsweise des Silikonstrumpfes, vorgesehen sein.

Der Schaft umfasst dann gemäß der Erfindung auch noch eine Vorrichtung zum Temperaturabbau Zumindest in Teilbereichen ist er dazu doppelwandig ausgebildet so dass PCM und Wärmetauschersystem praktisch im Schaft zu integrieren sind. Die Mittel zum Bewegen des Kühlmediums, also beispielsweise ein Ventilator oder eine kleine Pumpe, können dann sowohl für das Kühlvlies, als auch für den im festen Teil des Gegenstandes, also beispielsweise im Schaft integrierten, Wärmetauscher gemeinsam genutzt werden.

Die Mittel, durch die das Medium des Wärmetauschers mit den PCMs in Wärmeleitenden Kontakt kommt, umfassen auch eine Vorrichtung zur Erzeugung einer Strömung, beispielsweise Ventilator, Pumpe oder eine Öffnung im Schuh oder Sattel, durch die bei Bewegung Luft durch den Schuh und die Strömungsstrukturen gepumpt wird. Die Strömungsstrukturen bewirken, dass das Medium an den PCMs vorbeiströmt. Sie können auf der flexiblen Matrix mit den PCMs oder auch getrennt davon, also extern, vorgesehen sein. So können diese Strömungsstrukturen auch an der Innenseite des Schaftes einer Prothese, an der Innenseite eines Schuhs oder eines sonstigen starren Gegenstandes, der in Körperkontakt mit einem Lebewesen steht, vorgesehen sein. Die Mittel sind so gestaltet, dass das Medium eines Wärmetauschers durch sie in wärmeübertragenden Kontakt mit den PCMs des flexiblen Flächengebildes gebracht wird.

Es kann vorgesehen sein, dass Bereiche der flexiblen Matrix oder des Gegenstandes keine Vorrichtung zum Temperaturabbau umfassen, also nicht mit PCM-haltigen Bereichen oder Inseln versetzt sind und/oder dass in diesen Bereichen des Gegenstandes oder der flexiblen Matrix keine Strömungsstrukturen vorgesehen sind und sie entsprechend nicht von dem Medium umströmt werden.

Nach einer weiteren Ausführungsform umfasst das flexible Flächengebilde mehrere Lagen, also einen Stapel, wobei beispielsweise in miniaturisierter Form die Inseln mit PCMs und eine flexible Matrix sich abwechseln. Dabei können verschiedenste Eigenschaften des flexiblen Flächengebildes realisiert werden, insbesondere können auf der zum Körper zeigenden Seite des Stapels andere Oberflächeneigenschaften als auf der nach extern zeigenden Seite des Stapels realisiert sein. Das Material der flexiblen Matrix und auch die PCMs können innerhalb einer Lage und innerhalb des Stapels variieren.

Dabei kann beispielsweise ein Schichtaufbau wie folgt realisiert sein:
Flexible Matrix mit PCM-Bereichen,
darauf eine Lage mit Erhebungen, die Strömungskanäle für das Medium des Wärmetauschers bilden,
darauf wieder eine PCM-führende Lage und dann wieder eine Wärmetauschermediumführende Lage,
wobei die Flexibilität der Lagen und des Gesamtaufbaus trotz der Strukturierung möglichst erhalten bleibt oder sogar noch entsprechend den vorgesehenen Bewegungsabläufen, diese unterstützen kann.

Beispielsweise gibt es mehrlagige Silikonstrümpfe zur Weichteilstabilisierung am Körper, die hier als flexible Matrix dienen können. In diese werden dann die PCMs, beispielsweise in Form von flächigen kleinen Folienausschnitten, in diskreten Bereichen eingearbeitet, eingeschnitten oder aufgesetzt, so dass die mechanischen Eigenschaften wie Flexibilität und Reißfestigkeit der flexiblen Matrix im Wesentlichen erhalten bleiben. Die Einarbeitung der PCMs in die flexible Matrix richtet sich nach den jeweiligen Materialien, sowohl der Matrix als auch des PCMs, im Sinne der Erfindung soll aber jede Art der Einarbeitung der PCMs in eine flexible Matrix umfasst sein.

Neben Umgebungsluft und anderen gasförmigen Medien als Wärmetauschermedium kann beispielsweise auch ein flüssiges Wärmetauschermedium oder einfach Wasser vorgesehen sein.

Als Wärmetauschermedium dient vornehmlich die Umgebungsluft, allerdings kann der Wärmetauscherbetrieb sowohl offen als auch geschlossen geführt werden, wobei das Medium, das in Wärmekontakt mit den PCMs kommt, bevorzugt aber nicht notwendigerweise kontinuierlich regeneriert wird. Als "Regeneration" des Mediums wird vornehmlich verstanden, dass das Medium die vom Körper über das PCM aufgenommene Wärme wieder abgibt.

Beispielsweise umfasst der Wärmetauscher ein von PCM umgebenes Schlauchsystem, durch das Wasser oder Umgebungsluft geführt wird.

Besonders bevorzugt ist dabei, dass das Schlauchsystem selbst, also beispielsweise das Material aus dem der Schlauch ist, die PCMs umfasst.

Beispielsweise umfasst der Wärmetauscher auch einen Ringspalt eines Doppelwandigen Gegenstandes.

Nach einer weiteren Ausführungsform umfasst der Wärmetauscher Microlöcher oder Microspalte, durch die Umgebungsluft strömt oder gepumpt wird.

Als "Gegenstände, die in direktem Wärmekontakt zum Körper eines Lebewesens stehen", werden vorliegend beispielsweise Helme, Prothesen, Orthesen, Schuhe, Sättel, (von Fahrrädern sowie auf Pferden) Autositze, Handschuhe, alle Arten von Textilien, Surfanzüge, Tauchanzüge, Brillen, und ähnliches bezeichnet. Im Sinne der Erfindung soll alles umfasst sein, was Stauwärme des Körpers erzeugt.

Als Phasenwechselmaterial werden alle Arten von marktüblichen PCMs bezeichnet, die zur Latentwärmespeicherung fähig sind.

Unter Latentwärmespeicherung versteht man die Speicherung von Wärme in einem Material, welches einen Phasenübergang, vorwiegend fest - flüssig, erfährt (engl. Phase Change Material, PCM). Neben dem Phasenübergang fest-flüssig können prinzipiell auch fest-fest Phasenübergänge eingesetzt werden. Diese zeigen in der Regel jedoch weit geringere Speicherdichten.

Bei der Einspeicherung von Wärme in das Speichermaterial beginnt das Material bei Erreichen der Temperatur des Phasenübergangs zu Schmelzen und erhöht dann, trotz weiterer Einspeicherung von Wärme, seine Temperatur nicht bis das Material komplett geschmolzen ist. Erst dann tritt wieder eine Erhöhung der Temperatur auf.

Da für längere Zeit trotz Wärmezufuhr keine merkliche Temperaturerhöhung auftritt, nennt man die während des Phasenübergangs eingespeicherte Wärme "versteckte Wärme" oder auch "latente Wärme". Im Falle eines Phasenübergangs fest - flüssig ist die latente Wärme in der Schmelz- oder Kristallisationswärme des Speichermaterials enthalten.

Latentwärmespeicherung kann in verschiedenen Temperaturbereichen erfolgen. Je nach Anwendungsfall muss dazu ein Material mit geeigneter Temperatur des Phasenwechsels ausgewählt werden. Dabei werden, je nach Temperaturbereich, verschiedene Materialklassen eingesetzt.

Für den Bereich der Körperwärme werden im Sinne der Erfindung allgemein Paraffine oder Salzhydrate bzw. deren Modifikationen als PCMs vorgeschlagen.

Die PCMs liegen in Form von Compounds vor Ausführungsformen hierfür sind Paraffin-Polymer-Compounds, Salzhydrate, Salzhydrat-GraphitCompounds, Paraffin-Silikat-Compounds etc.

Paraffin-Polymer-Compounds können auch als Folie, Granulate, Schlauch oder andere Formkörper vorliegen. In einer weiteren Ausgestaltung können diese PCMs bzw. deren Modifikationen in entsprechende Trägerstrukturen, z. B. kapillare Fasersaugstrukturen eingearbeitet sein.

Weitere bevorzugte Varianten für PCM und PCM-Compounds sind folienverpackte PCMs.

Als Medium für den Wärmetauscher wird vorliegend bevorzugt Umgebungsluft und/oder

Wasser eingesetzt, so dass allein durch das Durchströmen des die PCMs umgebenden Leitungssystems mit Umgebungsluft oder Wasser die Vorrichtung zum Temperaturabbau funktioniert.

Das Medium kann einer Regeneration oder Kühlung unterzogen werden, indem es beispielsweise an ein mobiles Kühlaggregat angeschlossen ist.

Nach einer anderen Ausführungsform ist das Medium beispielsweise Wasser, das durch das Leitungssystem fließt und durch ein mitgeführtes Kühlaggregat wieder regeneriert wird.

Eine weitere Ausführungsform zum zusätzlichen Herunterkühlen besteht aus einem mit Flüssigkeit tränkbarem Material, z. B. Vlies, das z. B. mit Wasser oder anderen verdunstbaren Flüssigkeiten beladen werden kann. Dieses Vlies sorgt durch die Verdunstungskühlung der Flüssigkeit durch den vorbeiziehenden, erzwungenen Luftstrom für eine stark herabgesetzte Kühllufttemperatur.

Eine bevorzugte Ausführungsform des Verdunstungsvlieses ist außerhalb des Schaftes, z. B. auf der Ansaugseite des Lüfters.

Im Folgenden wird die Erfindung noch anhand ausgewählter Bei- spiele näher erläutert:
Figur 1 zeigt das Beispiel einer Bein- oder Armprothese oder Orthese mit einer Vorrichtung zum Temperaturabbau gemäß der vorliegenden Erfindung,
Figur 2 zeigt eine weitere Prothese, in der die Vorrichtung integriert ist und
Figur 3 schließlich zeigt den Querschnitt durch einen Schlauch, wobei ein Compound als Material des Schlauches eingesetzt ist.
Figur 4 zeigt die gleiche Darstellung wie Figur 1, wobei Verdunstungswärme die Regeneration der PCMs zumindest unterstützt.

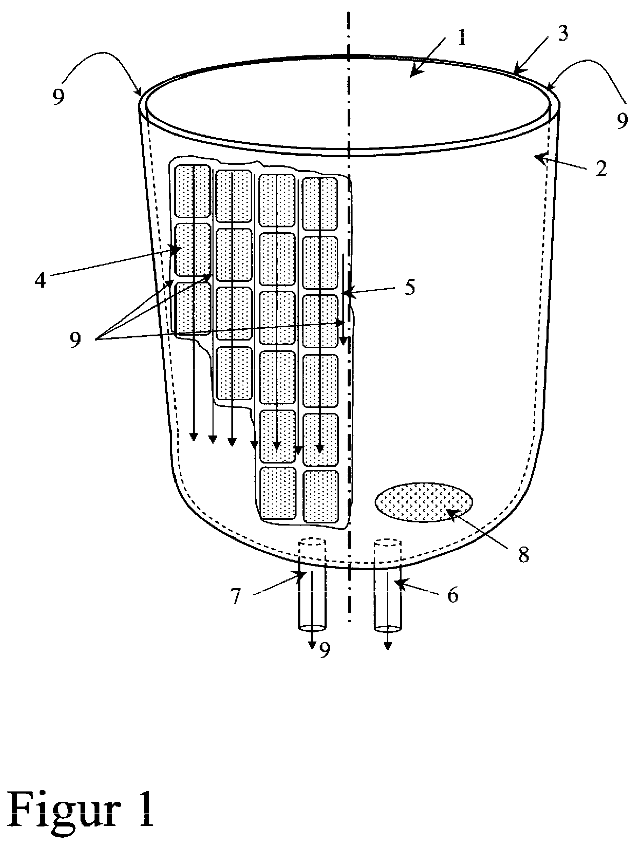

Figur 1 zeigt eine Prothese, in die im Fall einer Amputation ein so genannter Stumpf also der Rest des amputierten Gliedmaßes eingebracht wird. Normalerweise sind diese Prothese aus Harz, das einwandig ist.

Gemäß der hier gezeigten Ausführungsform nach der Erfindung wird anstelle der einwandigen Harzkonstruktion eine doppelwandige Konstruktion genommen, wobei die beiden Wände 1 und 2, also die Innenwand 1 und die Außenwand 2 eine Hohlraum definieren, der einen Ringspalt 3 bildet. Der Ringspalt 3 ist hier, wie gezeigt oben ganzseitig offen. Der Ringspalt kann jedoch auch nur partiell offen sein. Im Ringspalt 3 sind, wie hier gezeigt, PCM Module 4 als diskrete Päckchen eingearbeitet, die beispielsweise auf einer Oberfläche 5, beispielsweise eine Kautschukoberfläche, angeordnet sind. Unten an der Prothese ist zum einen das Absaugventil 6, das den Unterdruck 5 in der Prothese aufrechterhält und zum anderen zwischen den beiden Wänden 1 und 2 das Absaugventil 7, durch das Kühlmedium, das durch den Ringspalt 3 um die PCM Module 4 fließt, abgesaugt wird. Die Pfeile 9 bezeichnen den Weg des gasförmigen Kühlmediums durch den Ringspalt 3 um die PCM Module 4 herum. Das Ventil 7 ist beispielsweise an eine Pumpe, an einen Ventilator und/oder an ein externes Kühlaggregat (nicht gezeigt) angeschlossen, durch die/das die Luft oder das Kühlmedium durch den Ringspalt 3 hindurch geleitet, angesaugt, gepumpt und/oder gekühlt wird und wodurch die PCM Module Wärme abgeben, oder wodurch Feuchte aus der Orthese aufgenommen wird.

Der Wärmetauscherbetrieb wird hier demnach offen geführt, da beispielsweise einfach Umgebungsluft durch das Ventil 7 durch den offenen Ringspalt 3 hindurch angesaugt wird.

Nach einer bevorzugten Ausführungsform befindet sich an der Innenwand 1 der Prothese noch ein Temperatursensor, durch den die Innentemperatur feststellbar ist. Der Temperatursensor ist mit einer externen Regel- und Steuereinrichtung verbunden, die den Durchfluss und/oder die Temperatur des Kühlmediums regelt.

Nach der in Figur 1 gezeigten Ausführungsform der Erfindung sind noch Microlöcher 8 in der Außenwand 2 der Prothese vorgesehen, durch die weiteres Kühlmedium, beispielsweise in Form von Umgebungsluft, durch den Ringspalt geleitet wird. Die Microlöcher sind hier nur exemplarisch gezeigt, sie können natürlich beliebig angeordnet sein, sowohl in der Größe der Anordnung als auch in der Form.

Die PCM Module können als diskrete Päckchen oder Paraffin- Polymer-Compound-Schüttung vorliegen, sie können aber auch in jeder anderen beliebigen Form, und auch als einfache Schüttung in dem unten geschlossenen Ringspalt vorliegen. Schließlich kann die Innenwand 2 ganz oder teilweise aus einem Compound bestehen, ein PCM und einen Kunststoff in Form eines Polymers, der beispielsweise auch eine flexible Matrix enthält, umfassend.

Die PCM Module 4 können ebenfalls gemäß der Erfindung beliebig verteilt im Ringspalt 3 vorliegen. Beispielsweise wenn oben weniger Kühlung und unten mehr gebraucht wird, kann die Dichte der PCM Module über den Ringspalt 3 hinweg beliebig variieren. Die PCM Module können schließlich auch beweglich und/oder lösbar angeordnet sein, so dass Module verschoben oder zusätzliche Module hinzu und wieder weggenommen werden können.

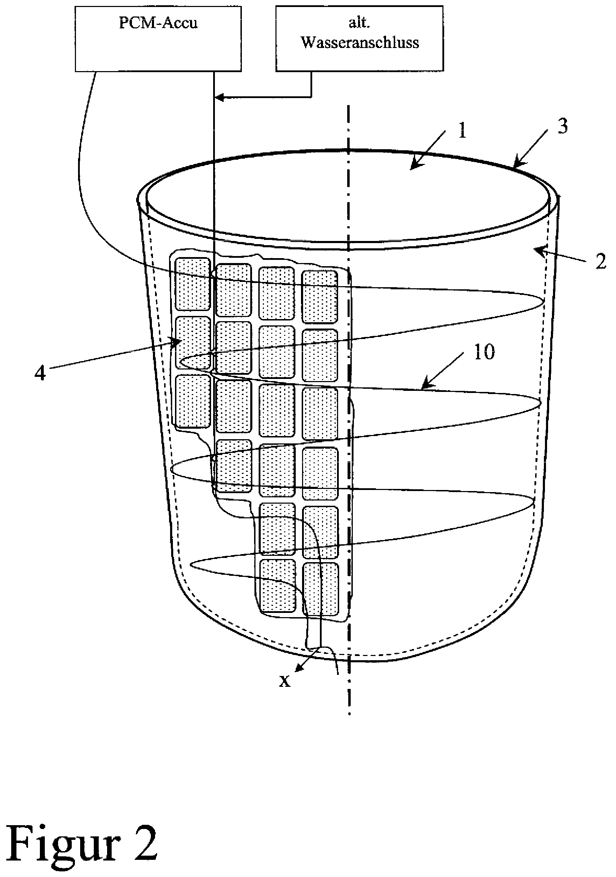

Figur 2 zeigt, wie Figur 1, das Beispiel einer Orthese oder Prothese. Wieder ist die doppelwandige Konstruktion mit Innenwand 1 und Außenwand 2 zu erkennen. Hier wird aber nicht, wie in der Figur 1, das Kühlmedium durch einen offenen Ringspalt 3 geführt, sondern es gibt eine Schlauchwicklung 10, die zwischen den PCM Modulen 4 gelegt ist und in der das Kühlmedium des Wärmetauschers fließt. Die Schlauchwicklung als Wärmetauscher ist nicht notwendigerweise ein geschlossenes System und kann beispielsweise mit Luft als Kühlmedium so betrieben werden, dass die Enden der durchgeblasenen Kühlluft im Innenraum der Orthese/Prothese enden. Andererseits kann als Kühlmedium auch ein flüssiges Medium, beispielsweise Wasser eingesetzt werden und der Wärmetauscher wird dann als geschlossenes System mit einer Rückführung der Schläuche an einen Wasseranschluss, einen weiteren Wärmetauscher oder ein Wasserkühlsystem (extern, nicht gezeigt) betrieben.

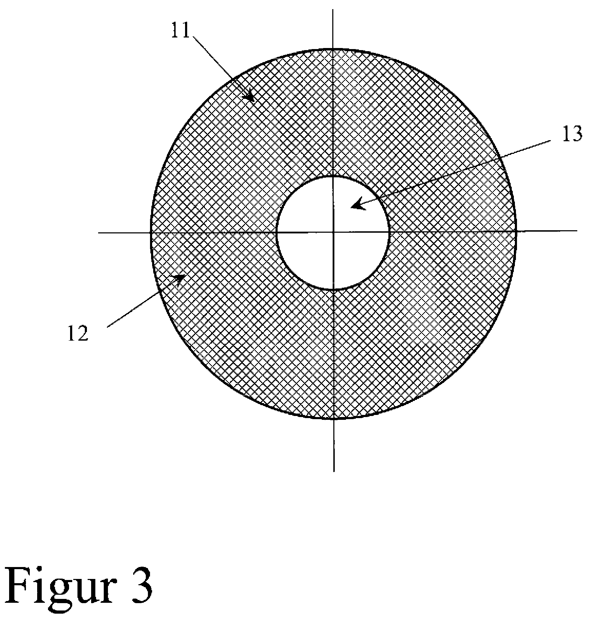

Figur 3 schließlich zeigt einen Querschnitt durch einen Schlauch 11, der gemäß der Erfindung zusammen mit einem doppelwandigen System, in dem PCM Module vorliegen, oder auch ohne weitere PCM Module einsetzbar ist. Der Schlauch 11 zeichnet sich dadurch aus, dass er einen Kanal 13 für das Kühlmedium bildet und der Schlauch 11 ein Compound mit PCM 12 führt, das z. B. ein PCM in einer Polymermatrix enthält.

Ein Schlauch wie in Figur 3 gezeigt, kann in verschiedenen Dicken hergestellt werden und in alle Arten von starren und flexiblen Gegenständen integriert werden. Die verschiedensten Anforderungen können hierbei erfüllt werden, weil Durchmesser der Schlauchwand, Innendurchmesser des Strömungsraumes und schließlich das Compound, in das das PCM eingearbeitet ist, frei wählbar sind.

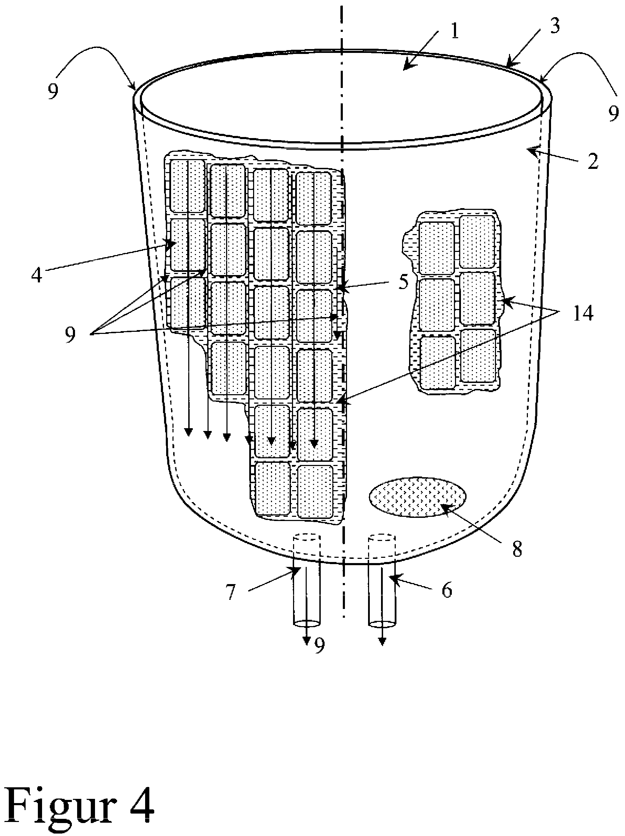

Figur 4 zeigt eine weitere Ausführungsform der Kälteerzeugung, in dem die beispielsweise über einen Lüfter geförderte Kühlluft gemäß den Pfeilen 9 im Schaft über ein mit z. B. Wasser getränktes Vlies 14 geführt wird und durch die Verdunstungskühlung der Flüssigkeit des Vlieses durch den vorbeiziehenden, erzwungenen Luftstrom für eine stark herabgesetzte Kühllufttemperatur sorgt.

Als Beispiel sei hier noch kurz die Anwendung in Schuhsohlen skizziert. Anstelle einer aufwändigen Pump- oder Ansaugvorrichtung kann hier einfach die Gehbewegung das Pumpen des Kühlmediums durch den Schlauch hindurch bewirken. Die Ausführungsform als PCM-haltiger Schlauch ist vielfältig einsetzbar, überall wo physische Bewegung des Körpers die externen Vorrichtungen zur Bewegung des Kühlmediums ersetzen, kann der Schlauch eingearbeitet werden.

Durch die Erfindung wird eine Vorrichtung aus PCM und einem aktiv kühlendem Wärmetauschersystem offenbart, dessen Anwendung bei Menschen und Tieren das bisherige Temperaturstauproblem zuverlässig löst. Das System kann in jeder Festigkeit realisiert werden also von der Integration in flexiblen Strümpfen bis zur Integration in Orthesenschäften. Dadurch ergeben sich die vielfältigsten Einsatzgebiete der beschriebenen Technik, die PCMs durch Module in Kühlmittelkanälen oder durch PCM-haltige Compounds, aus denen Schläuche gefertigt werden, in denen Kühlmedium fließen kann, über PCMs mit Wärmetauscher die Stauwärme des Körpers abzuführen.

## Patentansprüche

1. Doppelwandiger Prothesenschaft, der in einem direkten Wärmekontakt mit dem Körper eines Lebewesens steht, mit einer Innenwand (1) und einer Außenwand (2), die einen Hohlraum in Gestalt eines Ringspaltes (3) oder Strömungsstrukturen definieren und einem Wärmetauscher, der in dem Prothesenschaft integriert ist, wobei der Ringspalt (3) oder die Strömungsstrukturen von einem Kühlmedium durchflossen wird oder werden, **dadurch gekennzeichnet, dass** in dem Ringspalt (3) oder den Stömungsstrukturen ein Phasenwechselmaterial (PCM) angeordnet ist, das PCM als Compound ausgebildet ist und mit dem Wärmetauscher in Wärme übertragendem Kontakt steht.

2. Prothesenschaft nach Anspruch 1, **dadurch gekennzeichnet, dass** der Wärmetauscher als offenes System ausgebildet ist.

3. Prothesenschaft nach Anspruch 1 oder 2, **dadurch gekennzeichnet, dass** als Kühlmedium des Wärmetauschers Umgebungsluft eingesetzt wird.

4. Prothesenschaft nach einem der voranstehnden Ansprüche, **dadurch gekennzeichnet, dass** das PCM in Form von PCM-haltigen Modulen und/oder aus einer Schüttung von PCM-haltigen Partikel vorliegt.

5. Prothesenschaft nach einem der voranstehenden Ansprüche, **dadurch gekennzeichnet, dass** das PCM als Modifikation von Paraffin und/oder Salzhydrat vorliegt.

6. Prothesenschaft nach Anspruch 5, **dadurch gekennzeichnet, dass** das Compound in Form eines Schlauches bzw. mit PCM gefüllten Pads vorliegt.

7. Prothesenschaft nach einem der voranstehenden Ansprüche, **dadurch gekennzeichnet, dass** das PCM in eine Trägerstruktur eingearbeitet ist oder lösbar an dem Prothesenschaft befestigt ist.

8. Prothesenschaft nach einem der voranstehenden Ansprüche, **dadurch gekennzeichnet, dass** die Strömungsstrukturen in den PCM oder zwischen Inseln oder Bereichen mit PCM ausgebildet sind, durch die das Kühlmedium geleitet wird.

9. Prothesenschaft nach einem der voranstehenden Ansprüche, **dadurch gekennzeichnet, dass** der Wärmetauscher einen Ventilator aufweist.

10. Prothesenschaft nach einem der voranstehenden Ansprüche, **dadurch gekennzeichnet, dass** die PCM auf der Innenseite der Außenwand (2) oder auf der Außenseite der Innenwand (1) angeordnet sind oder die Innenwand (1) aus einem Compound mit einem PCM und einem Kunststoff in Form eines Polymers besteht.

11. Prothesenschaft nach einem der voranstehenden Ansprüche, **dadurch gekennzeichnet, dass** ein Absaugventil (6, 7) zur Aufrechterhaltung eines Unterdruckes an dem Prothesenschaft angeordnet ist.

12. Prothesenschaft nach einem der voranstehenden Ansprüche, **dadurch gekennzeichnet, dass** ein externes Kühlaggregat an dem Prothesenschaft angeschlossen ist, durch das das Kühlmedium geleitet wird.

13. Prothesenschaft nach einem der voranstehenden Ansprüche, **dadurch gekennzeichnet, dass** in der Außenwand (2) Microlöcher (8) vorgesehen sind.

14. Prothesenschaft nach einem der voranstehenden Ansprüche, **dadurch gekennzeichnet, dass** an der Innenwand (1) ein Temperatursensor angeordnet ist, der mit einer externen Regel- und Steuereinrichtung verbunden ist, die den Durchfluss und/oder die Temperatur des Kühlmediums regelt.

15. Prothesenschaft nach einem der voranstehenden Ansprüche, **dadurch gekennzeichnet, dass** die Innenwand (1) aus einem Silikon, Polyurethan, einem thermoplastischen Polymer, einem Copolymer oder Polyethylen besteht.

16. Prothesenschaft nach einem der Ansprüche 1 bis 14, **dadurch gekennzeichnet, dass** die Innenwand (1) als flexibler Silikonstrumpf zur Weichteilstabilisierung ausgebildet ist und im angelegten Zustand direkt auf der Haut aufliegt.

17. Prothesenschaft nach einem der voranstehenden Ansprüche, **dadurch gekennzeichnet, dass** die Strömungsstrukturen Kanäle oder Erhebungen mit verschiedenen Profilen wie Rillen, Noppen oder Pyramiden umfassen.

## Claims

1. Double-walled prosthesis socket in direct thermal contact with the body of a living being, having an inner wall (1) and an outer wall (2) which define a cavity in the form of an annular gap (3) or flow structures, and having a heat exchanger which is integrated in the prosthesis socket, wherein a coolant flows through the annular gap (3) or the flow structures, **characterized in that** a phase change material (PCM) is arranged in the annular gap (3) or the flow structures, and the PCM is configured as a compound and is in heat-transferring contact with the heat exchanger.

2. Prosthesis socket according to Claim 1, **characterized in that** the heat exchanger is designed as an open system.

3. Prosthesis socket according to Claim 1 or 2, **characterized in that** ambient air is used as the coolant of the heat exchanger.

4. Prosthesis socket according to one of the preceding claims, **characterized in that** the PCM is present in the form of PCM-containing modules and/or from a charge of PCM-containing particles.

5. Prosthesis socket according to one of the preceding claims, **characterized in that** the PCM is present as a modification of paraffin and/or salt hydrate.

6. Prosthesis socket according to Claim 5, **characterized in that** the compound is present in the form of a hose or PCM-filled pad.

7. Prosthesis socket according to one of the preceding claims, **characterized in that** the PCM is incorporated into a carrier structure or is fastened detachably to the prosthesis socket.

8. Prosthesis socket according to one of the preceding claims, **characterized in that** the flow structures are formed in the PCM or between islands or areas with PCM through which the coolant is conveyed.

9. Prosthesis socket according to one of the preceding claims, **characterized in that** the heat exchanger has a ventilator.

10. Prosthesis socket according to one of the preceding claims, **characterized in that** the PCMs are arranged on the inner face of the outer wall (2) or on the outer face of the inner wall (1), or the inner wall (1) is made of a compound with a PCM and a plastic in the form of a polymer.

11. Prosthesis socket according to one of the preceding claims, **characterized in that** a suction valve (6, 7) for maintaining an underpressure is arranged on the prosthesis socket.

12. Prosthesis socket according to one of the preceding claims, **characterized in that** an external cooling unit, through which the coolant is conveyed, is connected to the prosthesis socket.

13. Prosthesis socket according to one of the preceding claims, **characterized in that** micro-holes (8) are provided in the outer wall (2).

14. Prosthesis socket according to one of the preceding claims, **characterized in that** a temperature sensor is arranged on the inner wall (1) and is connected to an external regulation and control unit that regulates the flow and/or the temperature of the coolant.

15. Prosthesis socket according to one of the preceding claims, **characterized in that** the inner wall (1) is made from a silicone, polyurethane, a thermoplastic polymer, a copolymer or polyethylene.

16. Prosthesis socket according to one of Claims 1 to 14, **characterized in that** the inner wall (1) is designed as a flexible silicone stocking for stabilizing soft-tissue parts and bears directly on the skin in the applied state.

17. Prosthesis socket according to one of the preceding claims, **characterized in that** the flow structures comprise channels or elevations with different profiles such as grooves, knobs or pyramids.

## Revendications

1. Tige de prothèse à double paroi, qui se trouve dans un contact thermique direct avec le corps d'un être humain, comprenant une paroi intérieure (1) et une paroi extérieure (2), qui définissent une cavité sous la configuration d'un intervalle annulaire (3) ou des structures d'écoulement, et un échangeur de chaleur, qui est intégré dans la tige de prothèse, dans laquelle l'intervalle annulaire (3) ou les structures d'écoulement est/sont traversé(es) par l'écoulement d'un milieu de refroidissement, **caractérisée en ce qu'**un matériau à changement de phase (PCM) est agencé dans l'intervalle annulaire (3) ou dans les structures d'écoulement, ledit matériau PCM étant réalisé sous forme d'un composite et étant en contact avec l'échangeur de chaleur d'une manière qui assure le transfert de chaleur.

2. Tige de prothèse selon la revendication 1, **caractérisée en ce que** l'échangeur de chaleur est réalisé comme un système ouvert.

3. Tige de prothèse selon la revendication 1 ou 2, **caractérisée en ce que** l'on emploie de l'air environnant à titre de milieu de refroidissement de l'échangeur de chaleur.

4. Tige de prothèse selon l'une des revendications précédentes, **caractérisée en ce que** le matériau PCM se présente sous la forme de modules contenant du matériau PCM et/ou d'un tas de particules contenant du matériau PCM.

5. Tige de prothèse selon l'une des revendications précédentes, **caractérisée en ce que** le matériau PCM se présente sous la forme d'une modification de paraffine et/ou d'hydrate de sel.

6. Tige de prothèse selon la revendication 5, **caractérisée en ce que** le composite se présente sous la forme d'un tuyau ou d'un coussin rempli de matériau PCM.

7. Tige de prothèse selon l'une des revendications précédentes, **caractérisée en ce que** le matériau PCM est intégré dans une structure porteuse ou est fixé de façon détachable sur la tige de prothèse.

8. Tige de prothèse selon l'une des revendications précédentes, **caractérisée en ce que** les structures d'écoulement sont réalisées dans le matériau PCM ou bien entre des îles ou des zones avec du matériau PCM, à travers lesquelles le milieu de refroidissement est mené.

9. Tige de prothèse selon l'une des revendications précédentes, **caractérisée en ce que** l'échangeur de chaleur comprend un ventilateur.

10. Tige de prothèse selon l'une des revendications précédentes, **caractérisée en ce que** le matériau PCM est agencé sur la face intérieure de la paroi extérieure (2) ou sur la face extérieure de la paroi intérieure (1), ou bien la paroi intérieure (1) est constituée d'un composite avec un matériau PCM et une matière plastique sous la forme d'un polymère.

11. Tige de prothèse selon l'une des revendications précédentes, **caractérisée en ce qu'**une soupape d'aspiration (6), 7) destinée au maintien d'une dépression est agencée sur la tige de prothèse.

12. Tige de prothèse selon l'une des revendications précédentes, **caractérisée en ce qu'**un groupe de refroidissement externe est raccordé à la tige de prothèse, à travers lequel est mené le milieu de refroidissement.

13. Tige de prothèse selon l'une des revendications précédentes, **caractérisée en ce que** des micro-trous (8) sont prévus dans la paroi extérieure (2).

14. Tige de prothèse selon l'une des revendications précédentes, **caractérisée en ce qu'**un capteur de température est agencé sur la paroi intérieure (1), lequel est connecté à un dispositif de régulation et de commande externe, qui régule le débit et/ou la température du milieu de refroidissement.

15. Tige de prothèse selon l'une des revendications précédentes, **caractérisée en ce que** la paroi intérieure (1) est constituée de silicone, polyuréthane, polymère thermoplastique, copolymère ou polyéthylène.

16. Tige de prothèse selon l'une des revendications 1 à 14, **caractérisée en ce que** la paroi intérieure (1) est réalisée sous forme d'une chaussette en silicone flexible pour la stabilisation des parties molles, et s'applique directement sur la peau dans la situation montée.

17. Tige de prothèse selon l'une des revendications précédentes, **caractérisée en ce que** les structures d'écoulement incluent des canaux ou des reliefs avec différents profils, comme des rainures, des boutons ou des pyramides.
